# EUROPEAN PATENT SPECIFICATION

(11) **EP 0 613 368 B1**
(45) Date of publication and mention of the grant of the patent: **08.10.1997**
(21) Application number: 93913462.3
(22) Date of filing: 01.07.1993
(51) Int. Cl.: A61K 7/48

(54) **SKIN REGENERATING COSMETIC COMPOSITION**
HAUTREGENERIERENDE KOSMETISCHE ZUSAMMENSETZUNG
COMPOSITION COSMETIQUE DE REGENERATION CUTANEE

(30) Priority: 28.07.1992 HU 247092
(43) Date of publication of application: 07.09.1994
(73) Proprietor: CAOLA Cosmetics and Mixed Household Industry Rt., 1113 Budapest (HU)
(72) Inventor: SZALOKI, Erzsébet, H-4031 Debrecen (HU); SZABO, Eva, H-4032 Debrecen (HU); HEGEDÜS, Eva, H-4027 Debrecen (HU); JACSO, Erika, H-4031 Debrecen (HU); HANGACSI, Irén, H-4025 Debrecen (HU); KEREK, Kálmán, H-4032 Debrecen (HU); BERECZKI, Erzsébet, H-4032 Debrecen (HU); SAS, Erzsébet, H-4033 Debrecen (HU); SZABO, Tünde, H-4031 Debrecen (HU); KARANCSI, Erika, H-4031 Debrecen (HU); FABIAN, Agnes, H-1221 Budapest (HU); AGNI, Zsolt, H-4031 Debrecen (HU); PAPP, Gabriella, H-4034 Debrecen (HU); KOVACS, Aniko, H-4032 Debrecen (HU); NAGY, Ilona, H-4031 Debrecen (HU); JAKAB, Tünde, H-4029 Debrecen (HU); DEMKO, Gabriella, H-4032 Debrecen (HU); APAGYI, Erika, H-4032 Debrecen (HU)
(74) Representative: Eitle, Werner, Dipl.-Ing.
(86) International application number: PCT/HU93/00042
(87) International publication number: WO 94/02118

(56) References cited:
- CH-A- 660 454
- P.H. LIST, L. HOERHAMMER, "Hagers Handbuch der Pharmazeutischen Praxis", Volume 6, published 1979, by Springer Verlag (Berlin, Heidelberg, New York), pages 526, 527.
- P.H. LIST, L. HOERHAMMER, "Hagers Handbuch der Pharmazeutischen Praxis", Volume 6, part C, published 1979, by Springer Verlag (Berlin, Heidelberg, New York), pages 482-484.
- G.A. NOWAK, "Die Kosmetischen Praeparate", Volume 2, published 1984, by Verlag fuer chemische Industrie, H. Ziolkowsky KG. (Augsburg), pages 824-827.

## Description

The invention relates to skin regenerating cosmetic compositions comprising as active ingredient 3-60% by weight of extracts of Stellaria media and Viola tricolor.

The increasing number of environmental injuries, the polluted air, numerous flats not providing suitable micro-climate are causing various skin complaints in many people. In mild cases these appear in form of pathologically dry, inflammable, scurfy, itching skin deformations. When the skin problems are more serious dermatologists suggest the use of pharmaceutical compositions generally containing synthetic active ingredients.

In case of relatively mild deformations in the literature various paramedicinal or cosmetic composition are recommended to cease the symptoms.

Such a composition is described for example in the HU-PS 195.420 which contains extracts of St. John,s wort and marigold, tocopherol, collagen, allantoin, panthenol, jojoba oil, chamomile oil and chamomile extract.

According to HU-PS-195.915 the extract of different parts of the maize plant prepared with water or with water miscible pharmaceutically acceptable organic solvents is formulated together with the usual excipients, diluents or vehicles to skin regenerating and hydrating cosmetic composition.

A topical composition of similar effect is described in HU-PS 184.410, wich contains as active ingredient the aqueous or ethanolic extracts of drugs of species belonging to the Fagaceae and/or Chenopodicae and/or Rosaceae families.

CH-A-660454 and Hagers Handbuch der pharmaceutischen Praxis, Vol. 6, 1979, p. 482-484 both disclose the use of crude extracts of Viola tricolor in cosmetics.

With regard to the great variety of symptoms, the different sensitivity of the individuals, the potential allergic skin reactions, no composition is known which could be generally applied for the above group of symptoms.

The aim of our work was to prepare a new cosmetic composition which would enrich the choice of available compositions. Our further aim was to create a synergetic composition free of harmful side effects, comprising essentially natural active ingredients, suitable for the treatment of pathologically dry, inflammable, scurfy, itching skin deformations, eczemas and acnes in milder cases which do not require medical intervention.

We have surprisingly found that the above aims can be reached with a composition comprising as active ingredient extracts of chickweed (Stellaria media) and pansy (Viola tricolor).

The chickweed belongs to the family of Caryophyllaceae, its drug is the overground part of the plant collected in time of flowering. The main active ingredients of the drug are volatile oils, organic and inorganic salts, saponines. In case of internal use its antiphlogistic, blood-clearing, antipyretic, expectorating and antitussive effects are known. Externally its oily extract is suitable for the treatment of eruptions and certain types of psoriasis.

The pansy belongs to the family of Violaceae, its drug is he overground part of the plant, collected in time of flowering. The main active ingredients of the drug are violaquercetin, flavone glycoside, quercetin glycone, violanin anthocyan glycoside, sugars, organic acids, methyl

ester of salicylic acid, volatile oils. The tea made from the drug has expectorating, blood-clearing, hypotensive effects.

The present invention provides a skin regenerating cosmetic composition comprising:
ii) 3-60% by weight of an oily or aqueous and/or cosmetically acceptable organic solvent extract of parts of chickweed (stellaria media) and pansy (Viola tricolor) which contain crude drugs; and
(ii) 97-40% by weight of one or more cosmetically acceptable carriers and/or excipients and/or supplementary materials.

The compositions contain the extracts of the chickweed and pansy in a weight ratio from 7:1 to 1:7.

For preparing the compositions aqueous, aqueous ethanolic, aqueous propylene glycolic, cosmetic vaseline oily plant extracts can equally be used.

As vehicle the composition can contain artificial or natural fats, oils, fatty alcohols, esters of fatty acids and/or the derivatives thereof.

Suitable excipients are the thickening, epithelium regenerating, skin soothing, skin nurishing, tonic, antiseptic, moisturizing, solubilizing materials, stabilisers, preservatives, emulgeators, fillers, vitamins and fragrance.

The composition may contain supplementary components, such as light protecting materials, for example octyl-methoxy-cinnamate.

The plant extracts can be prepared in the usual way, among others by a process based on diffusion extraction (steeping, circulation extraction or counter-current extraction).

In these cases the parts of the plants are optionally dried, then crushed and extracted with 2-30 times the weight of extractive agent based on the amount of dried plants.

The extraction is carried out preferably at a temperature between 15°C and the boiling point of the extractive agent, by steeping the plant for 2-3 days or by countercurrent extraction for 1 hour.

In case of large scale embodiment it is advantageous to extract the plant at a higher temperature and for a shorter time, and if appropriate, combine the extraction with solvent recovery.

The plants can be extracted separately, and the extracts can be mixed when the composition is made, or the drugs can be blended in a determined ratio before the extraction.

The synergetic effect of the composition according to the invention was also examined. The details are described in the Comparative examples, and the results are summarized in the subsequent Table.

The solution according to our invention is illustrated by the following examples.

### Example 1

1 kg of dried, crushed drug of pansy was washed with water, then soaked in 2 kg of deionized water at room temperature for 2 days, with occasional stirring. After that the mixture was filtered.

### Example 2

1 kg of dried, crushed drug of pansy was washed with water, then extracted with 30 kg of water at 90°C for 30 minutes in an extractor. After cooling the extract was filtered. The solution obtained was tyndalled by heating it once a day to 80°C for 1 hour through 3 days.

### Example 3

The process described in Example 1 was followed, but for soaking water containing 30% by volume of propylene glycol was used.

### Example 4

1 kg of dried, crushed, washed drug of pansy was extracted with 10 kg of 70% by volume aqueous ethanol for 10 hours at 40 °C, under stirring. The extract obtained was filtered.

### Example 5

1 kg of dried, crushed, washed drug of pansy was extracted with 15 kg of cosmetic vaseline oil at 20°C for 2 days, stirring the mixture several times.
The plant pieces were removed by centrifuging, then the oily part was filtered.

### Example 6

The process described in Example 1 was followed, but instead of the drug of pansy the drug of chickweed was extracted.

### Example 7

The process described in Example 2 was followed, but instead of the drug of pansy the drug of chickweed was extracted.

### Example 8

The process described in Example 1 was followed, but instead of the drug of pansy the drug of chickweed was extracted with 2 kg of water containing 10% by volume propylene glycol.

### Example 9

The process described in Example 4 was followed, but instead of the drug of pansy the drug of chickweed was extracted with 50% by volume aqueous ethanol.

### Example 10

The process described in Example 5 was followed, but instead of the drug of pansy the drug of chickweed was extracted.

### Example 11

0,5 kg drug of pansy and 0,5 kg drug of chickweed was mixed, then thoroughly washed with water and extracted with 8 kg of water at 90°C for 50 minutes in a counter-current extractor. The solid part and liquid phase were separated by filtration.

### Example 12

| Emulsion composition: | |
|---|---|
| Aqueous pansy extract obtained in Example 1 | 2.62 g |
| Aqueous chickweed extract obtained in Example 6 | 0.38 g |
| Hydrogenated castor oil | 6.00 g |
| Avocado oil | 4.00 g |
| Maizen germ oil | 3.00 g |
| Vaseline oil | 14.00 g |
| Cetyl alcohol | 1.00 g |
| Propylene glycol | 4.00 g |
| Vitamin A | 0.05 g |
| Vitamin E | 0.07 g |
| Preservative | 0.10 g |
| Fragrance | 0.10 g |
| Deionized water | ad 100.00 g |

### Example 13

| Nurishing night cream composition: | |
|---|---|
| Aqueous pansy extract obtained in Example 2 | 7.50 g |
| Aqueous chickweed extract obtained in Example 7 | 52.50 g |
| Hydrogenated castor oil | 10.00 g |
| White vaseline | 5.00 g |
| Lanette® N (a mixture of sodium cetyl-stearyl sulfate and cetyl-stearyl alcohol, sold, HENKEL, DE) | 2.00 g |
| Avocado oil | 10.00 g |
| Vaseline oil | 4.00 g |
| Wheat germ oil | 5.00 g |
| Cholesterol | 2.60 g |
| Glycerol | 0.50 g |
| Vitamin A | 0.05 g |
| Vitamin E | 0.05 g |
| Preservative | 0.10 g |
| Fragrance | 0.10 g |
| Deionized water | ad 100.00 g |

### Example 14

| Night face cream composition: | |
|---|---|
| Oily pansy extract obtained in Example 5 | 10.00 g |
| Oily chickweed extract obtained in Example 10 | 10.00 g |
| Cutina® MD (a mixture of mono- and diglycerides of palmitic and stearic acids, HENKEL, DE) | 4.00 g |
| Stearol | 8.00 g |
| Emulgin® B1 (cetyl-stearyl-alcohol, HENKEL, DE) | 1.50 g |
| Emulgin® B2 (cetyl-stearyl-alcohol, HENKEL, DE) | 1.50 g |
| Eutanol® G (2-octyl-dodecanol, HENKEL, DE) | 15.00 g |
| Cutina® BW (partial glyceride and ester of long chain fatty acids, HENKEL, DE) | 2.00 g |
| Trietanolamine | 0.20 g |
| Deionized water | 47.80 g |

### Example 15

| Face tonic composition: | |
|---|---|
| Ethanolic pansy extract obtained in Example 4 | 25.00 g |
| Ethanolic chickweed extract obtained in Example 9 | 15.00 g |
| Glycerol | 2.00 g |
| Allantoin | 0.20 g |
| Cetiol® HE (polyetylene glycol-7-glyceryl-cocate, HENKEL, DE) | 2.00 g |
| Ethanol, 96% by volume | 5.20 g |
| Deionized water | ad 100.00 g |

### Example 16

| Liquid cleansing emulsion composition: | |
|---|---|
| Propylene glycolic pansy extract obtained in Example 3 | 9.00 g |
| Propylene glycolic chickweed extract obtained in Example 8 | 11.00 g |
| Texapon® EVR (sodium lauryl-ether sulfate with special ingredients, HENKEL, DE) | 40.00 g |
| Texapon® N 40 (sodium lauryl-ether sulfate, HENKEL, DE) | 40.00 g |

### Example 17

| Light protecting emulsion | |
|---|---|
| Aqueous extract obtained in Example 11 | 30.00 g |
| Glycerol-sorbitan-hydroxy-isostearate | 5.00 g |
| Isopropyl myristate | 5.00 g |
| Cyclic dimethyl-polysiloxane | 3.00 g |
| Polyoxyethylene stearyl-stearate | 4.00 g |
| Propylene glycol | 2.00 g |
| Etoxylated, hydrogenated castor oil | 0.50 g |
| Hydrogenated castor oil | 0.70 g |
| Fragrance | 0.10 g |
| Olive oil | 10.00 g |
| Octyl-methoxy-cinnanate | 0.20 g |
| Deionized water | ad 100.00 g |

### Example 18

| Body lotion | |
|---|---|
| Extract obtained in Example 3 | 2.00 g |
| Extract obtained in Example 8 | 2.00 g |
| Polyglyceryl-3-dioleate | 4.00 g |
| White vaseline | 10.00 g |
| White wax | 2.00 g |
| Paraffine, microcrystalline | 2.00 g |
| Alfa-bisabolol | 0.20 g |
| Dichloro-benzyl alcohol | 0.10 g |
| Cetearyl octanoate | 8.00 g |
| Magnesium sulfate heptahydrate | 0.70 g |
| Allantoin | 0.40 g |
| Imidazolidinyl urea | 0.30 g |
| Deionized water | ad 100.00 g |

### Example 19

| Face peeling masc composition: | |
|---|---|
| Aqueous extract obtained in Example 11 | 25.00 g |
| Cetyl-stearyl alcohol | 10.00 g |
| Paraffin oil | 10.00 g |
| Almond oil | 3.00 g |
| Myristyl-ethoxy-myristate | 2.00 g |
| Apricot kernel powder | 2.00 g |
| Allantoin | 0.20 g |
| Sepicide® HB (paraben and phenoxy-ethanol, SEPPIC, FR) | 0.30 g |
| Fragrance | 0.20 g |
| Deionized water | ad 100.00 g |

### Comparative example 1

The composition of Example 18 was prepared, but instead of pansy extract the same quantity of deionized water was used. The composition was examined on 25 women and 5 men aged from 30 to 55 years. The face skin of the treated persons were dry, slightly inflamed, scurfy and in some cases itching. The composition was applied twice a day for two weeks. After this period the effect was evaluated and the following results were obtained:
- in 13 cases no change was observed,
- in 7 cases the desquamation and itching was lessening,
- in 6 cases the desquamation, itching and inflammation was lessening, but the skin was dry,
- in 4 cases the complaints were ceased.

### Comparative example 2

The composition of Example 18 was prepared, but instead of chickweed extract the same quantity of deinoized water was used. The composition was examined on 24 women and 6 men aged from 32 to 58 years. The symptoms, the treatment and the way of evaluation was the same as described in Comparative example 1.

### Results:

- in 14 cases no change was observed,
- in 5 cases the inflammation and itching were lessening, but the skin was dry and scurfy,
- in 8 cases the inflammation and desquamation was significantly lessening, but the skin was still itching and dry,
- in 3 cases there were no complaints.

### Comparative example 3

The composition of Example 18 was prepared, but instead of the two plant extracts deionized water was used. The composition was examined on 27 women and 3 men aged from 33 to 57 years. The symptoms, the treatment and the way of evaluation was the same as described in Comparative example 1.

### Results:

- in 25 case the symptoms were not lessening,
- in 4 cases inflammation was hardly observable, dry ness and desqumation were slightly lessening,
- in 1 case all the symptoms were lessening in a medium degree.

### Comparative example 4

The composition of Example 18 was examined on 26 women and 4 men aged from 31 to 60 years. The symptoms, the treatment and the way of evaluation was the same as described in Comparative example 1.

### Results:

- in 1 case the symptoms were not lessening,
- in 2 cases inflammation, dryness, desquamation in a small degree could be well observed,
- in 4 cases the inflammation and itching in a small deg ree, the dryness and desquamation to a high extent were lessening,
- in 23 cases the treated persons became symptom-free.

**Table**

| Summary of the results obtained in Comparative examples 1-4 (%, based on the sample of 30 persons) | | | | |
|---|---|---|---|---|
| Example number | No effect was observed | Effect of the composition | | |
| | | weak | medium | good |
| 1 | 43 | 23 | 20 | 14 |
| 2 | 46 | 17 | 27 | 10 |
| 3 | 83 | 14 | 3 | - |
| 4 | 3 | 7 | 13 | 77 |

## Claims

1. A skin regenerating cosmetic composition comprising:
(i) 3-60% by weight of an oily or aqueous and/or cosmetically acceptable organic solvent extract of parts of chickweed (stellaria media) and pansy (Viola tricolor) which contain crude drugs; and
(ii) 97-40% by weight of one or more cosmetically acceptable carriers and/or excipients and/or supplementary materials.

2. A composition according to Claim 1, comprising the extracts of chickweed and pansy in a weight ratio 7:1 - 1:7.

3. A composition according to Claim 1 or Claim 2, comprising aqueous extracts of the plants.

4. A composition according to Claim 1 or Claim 2, comprising aqueous ethanolic extracts of the plants.

5. A composition according to Claim 1 or Claim 2, comprising aqueous propylene glycolic extracts of the plants.

6. A composition according to Claim 1 or Claim 2, comprising cosmetic vaseline oil extracts of the plants.

7. A composition according to Claim 1 or Claim 2, comprising artificial or natural fats, oils, fatty alcohols, fatty acid esters and/or derivatives thereof as a vehicle.

8. A composition according to Claim 1 or Claim 2, further comprising an excipient selected from one or more of a thickening material, an epithelium regenerater, a skin nurisher, an antiseptic, a tonic material, a stabilizer, a preservative, an emulsifier, a filler, a vitamin and a fragrance.

9. A composition according to Claim 1 or Claim 2, wherein the supplementary material is a light protecting material.

10. A composition according to Claim 9, wherein the light protecting material is octyl methoxy-cinnamate.

## Patentansprüche

1. Haut-regenerierende kosmetische Zusammensetzung, umfassend:
(i) 3 bis 60 Gew.% eines öligen oder wäßrigen und/oder kosmetisch verträglichen organischen Lösungsmittelextrakts von Teilen von Sternmire (Stellaria media) und Stiefmütterchen (Viola tricolor), die Rohdrogen enthalten, und
(ii) 97 bis 40 Gew.% eines oder mehrerer kosmetisch verträglicher Träger und/oder Exzipienten und/oder ergänzende Materialien.

2. Zusammensetzung nach Anspruch 1, enthaltend Extrakte von Sternmire und Stiefmütterchen in einem Gewichtsverhältnis von 7:1 bis 1:7.

3. Zusammensetzung nach Anspruch 1 oder 2, enthaltend wäßrige Extrakte der Pflanzen.

4. Zusammensetzung nach Anspruch 1 oder 2, enthaltend wäßrige ethanolische Extrakte der Pflanzen.

5. Zusammensetzung nach Anspruch 1 oder 2, enthaltend wäßrige propylenglycolische Extrakte der Pflanzen.

6. Zusammensetzung nach Anspruch 1 oder 2, enthaltend kosmetische Vaselineölextrakte der Pflanzen.

7. Zusammensetzung nach Anspruch 1 oder 2, enthaltend künstliche oder natürliche Fette, Öle, Fettalkohole, Fettsäureester und/oder Derivate davon als ein Vehikel.

8. Zusammensetzung nach Anspruch 1 oder 2, die ferner einen Exzipienten enthält, ausgewählt aus einem oder mehreren von einem Verdickungsmittel, einem Epithelregenerator, einem Hauternährungsmittel, einem Antiseptikum, einem tonischen Material, einem Stabilisator, einem Konservierungsmittel, einem Emulgator, einem Füllstoff, einem Vitamin und einem Duftstoff.

9. Zusammensetzung nach Anspruch 1 oder 2, worin das ergänzte Mittel ein Lichtschutzmittel ist.

10. Zusammensetzung nach Anspruch 9, worin das Lichtschutzmittel Octylmethoxycinnamat ist.

## Revendications

1. Composition cosmétique pour la régénération cutanée, comprenant :
(i) 3 à 60% en poids d'un extrait huileux ou aqueux et/ou dans un solvant organique acceptable du point de vue cosmétique de parties de mouron des oiseaux (Stellaria media) et de pensée (Viola tricolor) qui contiennent des médicaments bruts; et
(ii) 97 à 40% en poids d'un ou de plusieurs supports et/ou excipients et/ou matières supplémentaires acceptables du point de vue cosmétique.

2. Composition suivant la revendication 1, comprenant les extraits de mouron des oiseaux et de pensée selon un rapport en poids de 7:1 à 1:7.

3. Composition suivant les revendications 1 ou 2, comprenant des extraits aqueux des plantes.

4. Composition suivant les revendications 1 ou 2, comprenant des extraits aquo-éthanoliques des plantes.

5. Composition suivant les revendications 1 ou 2, comprenant des extraits aquo-propylène glycoliques des plantes.

6. Composition suivant les revendications 1 ou 2, comprenant des extraits des plantes dans une huile de vaseline cosmétique.

7. Composition suivant les revendications 1 ou 2, comprenant comme véhicule, des graisses, des huiles, des alcools gras, des esters d'acides gras et/ou des dérivés de ceux-ci, d'origine naturelle ou synthétique.

8. Composition suivant les revendications 1 ou 2, comprenant de plus un excipient choisi parmi un ou plusieurs composés tels qu'une substance épaississante, un régénérateur d'épithélium, un composé nourrissant la peau, un antiseptique, une matière tonique, un stabilisant, un conservateur, un émulsifiant, une charge, une vitamine et un arôme.

9. Composition suivant les revendications 1 ou 2, dans laquelle la matière supplémentaire est une matière protectrice contre la lumière.

10. Composition suivant la revendication 9, dans laquelle la matière protectrice contre la lumière est du méthoxycinnamate d'octyle.
